(19) Europäisches Patentamt
European Patent Office
Office européen des brevets

(11) Veröffentlichungsnummer : **0 358 016 B1**

(12) # EUROPÄISCHE PATENTSCHRIFT

(45) Veröffentlichungstag der Patentschrift :
**21.10.92 Patentblatt 92/43**

(51) Int. Cl.$^5$ : **C07D 401/12,** C08K 5/3447

(21) Anmeldenummer : **89115310.8**

(22) Anmeldetag : **19.08.89**

(54) **2,6-Polyalkylpiperidinsubstituierte Benzimidazol-2-carbonsäureanilide und deren Verwendung zum Stabilisieren von organischem Material.**

(30) Priorität : **23.08.88 DE 3828536**

(43) Veröffentlichungstag der Anmeldung :
**14.03.90 Patentblatt 90/11**

(45) Bekanntmachung des Hinweises auf die Patenterteilung :
**21.10.92 Patentblatt 92/43**

(84) Benannte Vertragsstaaten :
**BE CH DE ES FR GB IT LI NL**

(56) Entgegenhaltungen :
**EP-A- 0 284 828**

(73) Patentinhaber : **BASF Aktiengesellschaft**
**Carl-Bosch-Strasse 38**
**W-6700 Ludwigshafen (DE)**

(72) Erfinder : **Aumueller, Alexander, Dr.**
**An der Marlach 5**
**W-6705 Deidesheim (DE)**
Erfinder : **Spang, Peter, Dr.**
**Zur Audell 43**
**W-6670 St. Ingbert (DE)**
Erfinder : **Neumann, Peter, Dr.**
**Poststrasse 28**
**W-6800 Mannheim 31 (DE)**
Erfinder : **Trauth, Hubert**
**Milanstrasse 5**
**W-6724 Dudenhofen (DE)**

EP 0 358 016 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, daß Polyalkylpiperidinderivate organische Polymere vor Zerstörung durch Licht und Wärme schützen.

Unbefriedigend ist bei den Stabilisatoren des Standes der Technik oft deren Verträglichkeit mit Polyolefinen und anderen Kunststoffen, die Dauer der Schutzwirkung, deren Flüchtigkeit und deren thermische Zersetzung beim Einarbeiten in die Polymeren bei erhöhter Temperatur.

Der Erfindung lag die Aufgabe zugrunde, neue Stabilisatoren zur Verfügung zu stellen, welche die vorstehenden Nachteile nicht aufweisen.

Die Aufgabe wird gelöst mit einer molekularen Kombination aus Benzimidazolcarbonsäureaniliden und Polyalkylpiperidinderivaten. Dementsprechend betrifft die Erfindung polyalkylpiperidinsubstituierte Benzimidazolcarbonsäureanilide der allgemeinen Formel (I)

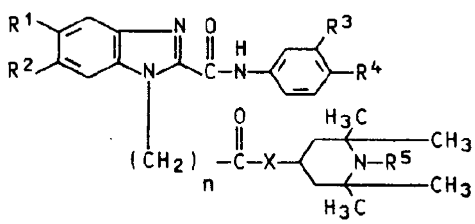

(I),

in der

$R^1$ und $R^2$ unabhängig voneinander für Wasserstoff, Chlor, Brom, $C_1$-$C_4$-Alkyl, $C_1$-$C_4$-Alkoxy, gegebenenfalls durch $C_1$- bis $C_4$-Alkyl oder $C_1$-bis $C_4$-Alkoxy substituiertes Phenyl oder $C_7$- bis $C_9$-Phenylalkyl,

$R^3$ und $R^4$ unabhängig voneinander für Wasserstoff, $C_1$- bis $C_{18}$-Alkyl, durch Sauerstoff ein- oder mehrfach unterbrochenes $C_3$- bis $C_{18}$-Alkyl, $C_1$- bis $C_{18}$-Alkoxy, durch Sauerstoff ein- oder mehrfach unterbrochenes $C_4$- bis $C_{18}$-Alkoxy, gegebenenfalls durch $C_1$- bis $C_4$-Alkyl oder $C_1$- bis $C_4$-Alkoxy substituiertes Phenyl, Phenyl-$C_1$-$C_8$-alkoxy, $C_1$- bis $C_{12}$-Alkanoylamino, Benzoylamino, $C_2$- bis $C_{12}$-Alkanoyloxy oder Benzoyloxy,

n für 0, 1, 2, 3, 4 oder 5,

X für -O-, 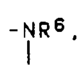, 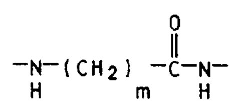, 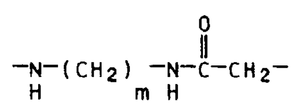 oder

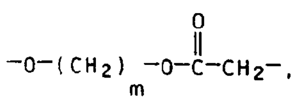,

$R^5$ für Wasserstoff, $C_1$- bis $C_8$-Alkyl, $C_7$- bis $C_{10}$-Phenylalkyl, Cyanmethyl, Aminoethyl, Hydroxyethyl, $C_1$- bis $C_8$-Alkylcarbonyl, Formyl oder Benzoyl,

$R^6$ für Wasserstoff, $C_1$- bis $C_8$-Alkyl, $C_7$- bis $C_{10}$-Phenylalkyl oder Phenyl und

m für eine Zahl von 2 bis 8 stehen,

sowie die Säureadditionsalze und Hydrate dieser Verbindungen.

Die Verbindungen (I) eignen sich sehr gut zum Stabilisieren von organischem Material, speziell von Kunststoffen, gegen den Abbau durch Licht und Wärme. Sie sind auch wirksam als Metalldesaktivator. Bevorzugt werden die neuen Verbindungen zum Stabilisieren von Polyolefinen, insbesondere von Ethylen- oder Propylenpolymerisaten, sowie von Polyurethanen und ABS verwendet. Weiterhin wird (I) bevorzugt zur Stabilisierung von Polyamiden verwendet. Den zu stabilisierenden Kunststoffen werden die Verbindungen (I) in einer Menge von 0,01 bis 5 Gew.%, vorzugsweise von 0,02 bis 1 Gew.%, bezogen auf das Polymere, vor, während oder nach der Polymerbildung zugesetzt.

Alkylreste für $R^1$ und $R^2$ sind beispielsweise Methyl, Ethyl, n-Propyl, iso-Propyl, n-Butyl, iso-Butyl oder t-Butyl.

Alkoxyreste für $R^1$ und $R^2$ sind beispielsweise Methoxy, Ethoxy, n-Propoxy, i-Propoxy und n-Butoxy.

Weitere Reste $R^1$ und $R^2$ können beispielsweise sein: Phenyl, Tolyl, Methoxyphenyl, Ethoxyphenyl, Benzyl, Phenylethyl, Phenylpropyl und insbesondere Wasserstoff.

Alkylreste für $R^3$ und $R^4$ sind beispielsweise Methyl, Ethyl, Propyl, Butyl, iso-Propyl, iso-Butyl, tertiär-Butyl,

Pentyl, iso-Pentyl, Hexyl, Heptyl, Octyl, Nonyl, Decyl, Undecyl, Dodecyl und Octadecyl.

Alkoxyreste für $R^3$ und $R^4$ können beispielsweise sein: Methoxy, Ethoxy, n-Propoxy, iso-Propoxy, n-Butoxy, iso-Butoxy, tertiär-Butoxy, Pentoxy, Hexoxy, 2-Ethyl-hexoxy, Heptyloxy, Octyloxy, Dodecyloxy und Octadecyloxy.

Gegebenenfalls substituierte Phenylreste für $R^3$ und $R^4$ sind z.B.: Phenyl, Tolyl, Methoxyphenyl, Ethoxyphenyl, Propoxyphenyl, Butoxyphenyl, Ethylphenyl, Propylphenyl, Butylphenyl, iso-Propylphenyl, iso-Butylphenyl, tert-Butylphenyl, iso-Propoxyphenyl und iso-Butoxyphenyl.

Als Phenylalkoxyreste kommen für $R^3$ und $R^4$ z.B. in Betracht: Benzyloxy, Phenylethoxy, Phenylpropoxy, Phenylbutoxy oder Phenylnonyloxy.

$R^3$ und $R^4$ können auch Phenoxy sein.

Als Alkanoylaminoreste sind für $R^3$ und $R^4$ z.B. zu nennen: Formylamino, Acetylamino, Propionylamino, Butanoylamino, iso-Butanoylamino, tertiär-Butanoylamino, Pentanoylamino, Hexanoylamino, 2-Ethylhexanoylamino, Heptanoylamino, Octanylamino, Nonanoylamino, Decanoylamino, Dodecanoylamino, Octadecanoylamino und Benzoylamino.

Alkanoyloxyreste für $R^3$ und $R^4$ sind z.B.: Acetyloxy, Propionyloxy, Butanoyloxy, iso-Butanoyloxy, tertiär-Butanoyloxy, Pentanoyloxy, Hexanoyloxy, 2-Ethylhexanoyloxy, Heptanoyloxy, Octanoyloxy, Nonanoyloxy, Decanoyloxy, Dodecanoyloxy, Octadecanoyloxy und Benzoyloxy.

Bevorzugt sind Verbindungen der Formel (I), bei denen $R^3$ für Wasserstoff und $R^4$ für Wasserstoff, Methyl, Ethyl, Propyl, Methoxy, Propoxy, Ethoxy, Butoxy, Phenoxy, Acetylamino oder Propionylamino steht.

Besonders bevorzugt sind Verbindungen der allgemeinen Formel (I), in denen

$R^3$ für Wasserstoff und

$R^4$ für Wasserstoff oder Ethoxy stehen.

n ist 0, 1, 2, 3, 4 oder 5, vorzugsweise 1 oder 2,

X kann neben Sauerstoff z.B.

$-\overset{|}{N}H$, $-\overset{|}{N}$-Methyl, $-\overset{|}{N}$-Ethyl, $-\overset{|}{N}$-Propyl, $-\overset{|}{N}$-Butyl, $-\overset{|}{N}$-Pentyl,

$-\overset{|}{N}$-Hexyl, $-\overset{|}{N}$-Heptyl, $-\overset{|}{N}$-Octyl, $-\overset{|}{N}$-Phenyl, $-\underset{H}{N}-(CH_2)_m-\overset{O}{\overset{\|}{C}}-\underset{H}{N}-$ ,

$-\underset{H}{N}-(CH_2)_m-\underset{H}{N}-\overset{O}{\overset{\|}{C}}-CH_2-$ oder $-O-(CH_2)_m-O-\overset{O}{\overset{\|}{C}}-CH_2-$ sein, worin

m eine ganze Zahl zwischen 2 und 8, vorzugsweise 2 ist.

Für X ist >NH oder

$$-HN-CH_2CH_2-\overset{O}{\overset{\|}{C}}-NH-$$

besonders bevorzugt.

Für $R^5$ sind z.B. im einzelnen zu nennen: Wasserstoff, Methyl, Ethyl, Propyl, Butyl, Pentyl, Hexyl, Heptyl, Octyl, Benzyl, Phenylethyl, Phenylpropyl, Phenylbutyl, Cyanmethyl, Aminoethyl, Hydroxyethyl, Formyl, Acetyl, Propionyl, Butanoyl, Pentanoyl, Hexanoyl, Heptanoyl oder Octanoyl.

Vorzugsweise steht $R^5$ für Methyl und insbesondere für Wasserstoff.

Die Verbindungen der allgemeinen Formel (I) lassen sich beispielsweise durch Umsetzen von Verbindungen der allgemeinen Formel (II), in der R ein niederer Alkylrest z.B. Methyl oder Ethyl ist, mit Verbindungen der allgemeinen Formel (III) in an sich bekannter Weise herstellen:

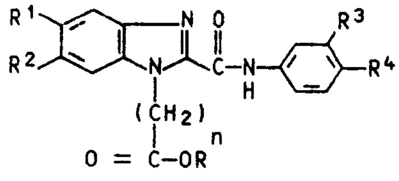

(II)

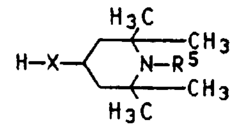

(III)

Verbindungen der allgemeinen Formel (II) können z.B. durch Umsetzen von Verbindungen der allgemeinen Formel IV mit Acrylestern, Halogencarbonsäureestern oder Halogencarbonsäuren und anschließender Veresterung hergestellt werden.

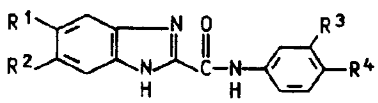

(IV)

Die Herstellung der Verbindungen der allgemeinen Formel (IV) ist z.B. in der EP-A-0 284 828 beschrieben.

Verbindungen der allgemeinen Formel (I) mit $R^5$ = H können nach an sich bekannten Verfahren, wie Alkylierung, Acylierung, reduktive Aminierung, Cyanmethylierung oder Hydroxyethylierung in Verbindungen der allgemeinen Formel (I) mit $R^5$ ungleich H überführt werden.

Die erfindungsgemäßen Verbindungen können in Form der freien Basen oder als Salze vorliegen. Geeignete Anionen stammen z. B. von anorganischen Säuren und insbesondere von organischen Carbonsäuren sowie organischen Sulfonsäuren.

Als anorganische Anionen sind z. B. Chlorid, Bromid, Sulfat, Methosulfat, Tetrafluoroborat, Phosphat und Rhodanid zu nennen.

Als Carbonsäureanionen kommen z. B. Formiat, Acetat, Propionat, Hexanoat, Cyclohexanoat, Lactat, Stearat, Dodecylbenzoat, Benzoat, Acrylat, Methacrylat, Citrat, Malonat oder Succinat sowie Anionen von Polycarbonsäuren mit bis zu etwa 3000 COOH-Gruppen in Betracht.

Sulfonsäure-Anionen sind z.B. Benzolsulfonat oder Tosylat.

Das Vermischen der erfindungsgemäßen Verbindungen (I) mit den Kunststoffen kann in allen bekannten Vorrichtungen und nach den bekannten Methoden zum Einmischen von Stabilisierungsmitteln oder anderen Zusätzen in Polymere erfolgen.

Die mit den erfindungsgemäßen Verbindungen stabilisierten Kunststoffe können gegebenenfalls noch weitere Additive enthalten, z.B. Antioxidantien, Lichtstabilisierungsmittel, Metalldesaktivatoren, antistatische Mittel, flammhemmende Mittel, Pigmente und/oder Füllstoffe.

Antioxidantien und Lichtstabilisatoren, die den Kunststoffen neben den erfindungsgemäßen Verbindungen zugesetzt werden können, sind z.B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-]β(3,5-di-tert.-butyl-4-hydroxyphenyl)-propionyloxyethyl]-isocyanurat, 1,3,5-Tris-(2,6,dimethyl-3-hydroxy-4-tert.-butylbenzyl)-isocyanurat und Pentaerythrit-tetrakis-[-(3,5-di-tert.-butyl-4-hydroxy-phenyl)-propionat] erwähnt.

Als phorphorhaltige Antioxidantien kommen beispielsweise Tris-(nonylphenyl-phosphit, Distearylpentaerythritdiphosphit, Tris-(2,4-di-tert.-butyl-phenyl)-phoshit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4'-biphenylendiphosphit in Betracht.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat) und Pentaerythrittetrakis-(β-laurylthiopropionat) und Pentaerythrittetrakis-(β-lauryl-thiopropionat) und Pentaerythrittetrakis-(β-hexylthiopropionat) genannt.

Weitere Antioxidantien und Lichtstabilisatoren, die zusammen mit den erfindungsgemäßen Verbindungen verwendet werden können, sind z.B. 2-(2'-Hydroxyphenyl)-benztriazole, 2-Hydroxybenzophenone, Arylester von Hydroxybenzoesäuren, α-Cyanozimtsäurederivate, Nickelverbindungen oder Oxalsäuredianilide.

Als organische Polymere, die durch die erfindungsgemäßen Verbindungen stabilisiert werden können, seien beispielsweise genannt:
Polymere von Mono- und Diolefinen, wie z. B. Polyethylen niedriger oder hoher Dichte, lineares Polybuten-1, Polyisopren, Polybutadien sowie Copolymerisate von Mono- oder Diolefinen oder Mischungen der genannten Polymeren;
Copolymerisate von Mono- oder Diolefinen mit anderen Vinylmonomeren wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;
Polystyrol;
Copolymere von Styrol oder α-Methylstyrol mit Dienen oder Acrylderivaten, wie z. B. Styrol-Butadien, Styrol-Acrylnitril, Styrol-EThylmethacrylat, Styrol-Butadien-Ethylacrylat, Styrol-Acrylnitril-Methacrylat;
ABS-, MBS- oder ähnliche Polymere;
Halogenhaltige Polymere, wie z. B. Polyvinylchlorid, Polyvinylfluorid, Polyvinylidenfluorid sowie deren Copolymere;
Polymere die sich von α, β-ungesättigten Säuren und deren Derivaten ableiten, wie Polyacrylate, Polymethacrylate, Polyacrylamide und Polyacrylnitrile;
Polymere, die sich un ungesättigten Alkoholen und Aminen bzw. von deren Acrylderivaten oder Acetalen ableiten, z. B. Polyvinylalkohol und Polyvinylacetat;
Polyurethane, Polyamide, Polyharnstoffe, Polyester, Polycarbonate, Polysulfone, Polyethersulfone und Polyetherketone.

Weiterhin können mit den erfindungsgemäßen Verbindungen Lacküberzuge stabilisiert werden, z. B. Industrielackierungen. Unter diesen sind Einbrennlackierungen, unter diesen wiederum Fahrzeuglackierungen, vorzugsweise Zweischichtlackierungen, besonders hervorzuheben.

Auch hier können zusätzlich die bereits genannten Antioxidatien und Lichtschutzmittel mitverwendet werden.

Die erfindungsgemäßen Verbindungen können in fester oder gelöster Form dem Lack zugesetzt werden. Ihr gute Löslichkeit in Lacksystemen ist dabei von besonderem Vorteil.

Die Erfindung wird durch die nachstehenden Beispiele weiter erläutert.

Beispiel 1

a) 169 g 4'-Ethoxy-benzimidazol-2-carbonsäureanilid, 99,4 g Kaliumcarbonat und 87 g Chloressigsäuremethylester wurden in 400 ml Dimethylformamid 75 Minuten bei 90 - 95 °C gerührt. Die heiße Reaktionsmischung wurde abesaugt, mit 150 ml heißem Dimethylformamid nachgewaschen und die Lösung mit Aktivkohle behandelt. Nach dem Abfiltrieren wurde die klare Lösung in ca. 4 l Methanol gegossen und 1 Stunde nachgerührt. Der ausgefallene Niederschlag wurde abgesaugt, mit Methanol gewaschen und getrocknet. Man erhielt 125 g der Verbindung der Formel

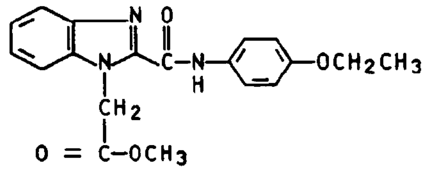

als farblosen Feststoff vom Schmp.: 140 - 141 °C.

Ber.: C 64,6 H 5,4 N 11,9 O 18,1
Gef.: C 64,6 H 5,6 N 11,9 O 18,0

b) 125 g des Produkts aus a) und 200 ml 2,2,6,6-Tetramethyl-4-amino-piperidin wurden 5 Stunden auf 130 - 135 °C erhitzt. Die Reaktionsmischung wurde heiß in 3,5 l Wasser eingerührt, der ausgefallene Niederschlag wurde abgesaugt, mit 500 ml Wasser gewaschen und getrocknet. Nach Umkristallisation aus Acetonitril erhielt man 80,2 g der Verbindung der Formel

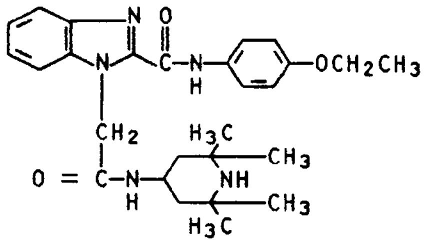

als farblosen Feststoff vom Schmp.: 213 - 215 °C.
Ber.: C 67,9 H 7,4 N 14,5 O 10,0
Gef.: C 67,8, H 7,6 N 14,5 O 10,2

Beispiel 2

a) 71,3 g Benzimidazol-2-carbonsäureanilid, 50 g Kaliumcarbonat und 66,8 g Bromessigsäureethylester wurden in 200 ml Dimethylformamid 1,5 Stunden auf 90 - 95 °C erhitzt. Die Reaktionsmischung wurde heiß filtriert, der Rückstand mit 80 ml heißem Dimethylformamid gewaschen. Die vereinigten Filtrate wurden in 2,5 l Ethanol gegossen. Der ausgefallene Niederschlag wurde abgesaugt, mit 200 ml kaltem Ethanol gewaschen und bei 90 °C getrocknet. Man erhielt 39,5 g der Verbindung der Formel

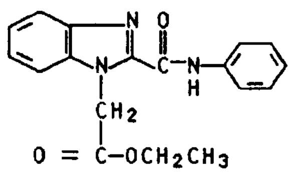

als farblosen Feststoff vom Schmp.: 135 - 138 °C.
Ber.: C 66,9 H 5,3 N 13,0 O 14,9
Gef.: C 67,2 H 5,5 N 12,9 O 14,5

b) 16,1 g des Produktes aus 2a) und 100 ml 2,2,6,6-Tetramethyl-4-amino-piperidin wurden 5 Stunden auf 110 - 120 °C und anschließend 4 Stunden auf 160 - 175°C erhitzt.

Anschließend wurde die Reaktionsmischung in 800 ml Wasser eingerührt, der ausgefallene Niederschlag wurde abgesaugt, mit Wasser gewaschen und getrocknet. Nach Umkristallisation aus Acetonitril erhielt man 7,3 g der Verbindung der Formel

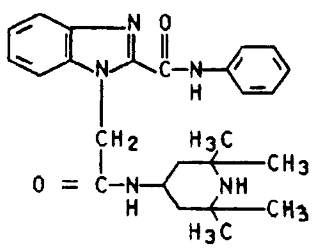

als farblosen Feststoff vom Schmp.: 220 - 225 °C.
Ber.: C 69,3 H 7,2 N 16,1 O 7,4
Gef.: C 69,5 H 7,0 N 15,5 O 8,1

Beispiel 3

a) 169 g 4′-Ethoxy-benzimidazol-2-carbonsäureanilid, 99,4 g Kaliumcarbonat und 98 g 3-Chlorpropionsäuremethylester wurden in 400 ml Dimethylformamid 2 Stunden auf 90 - 95 °C erhitzt. Die Aufarbeitung erfolgte wie in Beispiel 1. Die Fällung wurde in 4 l Methanol und 1000 g Eis durchgeführt. Nach Absaugen, Waschen und Trocknen erhielt man 175,9 g der Verbindung der Formel

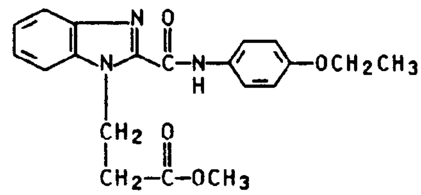

als farblosen Feststoff vom Schmp.: 121 - 122 °C.
Ber.: C 65,4 H 5,7 N 11,4 O 17,4
Gef.: C 65,5 H 5,8 N 11,3 O 17,5

b) 36,7 g des Produktes aus 3a) und 150 ml 2,2,6,6-Tetramethyl-4-amino-piperidin wurden 6 Stunden auf 130 - 135 °C erhitzt. Nach Zugabe von 2 ml einer 30 %igen methanolischen Natriummethylatlösung wurde weitere 3 Stunden auf 130 - 135°C erhitzt. Ca. 80 % des überschüssigen Amins wurden im Wasserstrahlvakuum abdestilliert, der Rückstand wurde in 170 ml Methanol gelöst, unlösliche Bestandteile wurden abfiltriert und die Lösung auf 600 ml Wasser gegeben. Der Niederschlag wurde abgesaugt und getrocknet. Nach Umkristallisation aus Toluol erhielt man 30,4 g der Verbindung der Formel

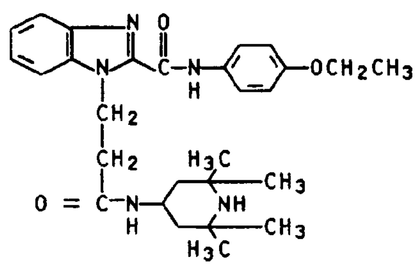

als farblosen Feststoff vom Schmp.: 144 - 147 °C.
Ber.: C 68,4 H 7,6 N 14,2 O 9,8
Gef.: C 68,8 H 7,5 N 13,5 O 9,8

Beispiel 4

28,3 g des Produktes aus Beispiel 1 und 23,0 g β-Alanin-2,2,6,6-tetramethyl-4-piperidinylamid wurden in 200 ml Ethanol unter Zusatz von 36,0 g 30 %iger methanolischer Natriummethylatlösung 4,5 h zum Sieden erhitzt. Das Lösungsmittel wurde im Vakuum abdestilliert, der Rückstand mit Wasser verrührt, abgesaugt, mit Wasser gewaschen und aus Acetonitril umkristallisiert.

Man erhielt 5,8 g der Verbindung der Formel

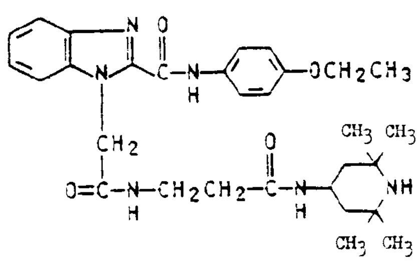

als farblosen Feststoff vom Schmp. 230°C.
Ber.: C 65,7 H 7,3 O 11,7
Gef.: C 65,4 H 7,8 O 12,4

**Patentansprüche**

1. Benzimidazolcarbonsäureanilide der allgemeinen Formel (I)

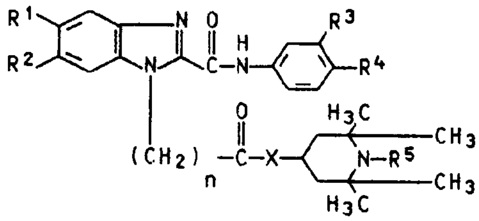

(I),

in der

$R^1$ und $R^2$ unabhängig voneinander für Wasserstoff, Chlor, Brom, $C_1$-$C_4$-Alkyl, $C_1$-$C_4$-Alkoxy, gegebenenfalls durch $C_1$- bis $C_4$-Alkyl oder $C_1$-bis $C_4$-Alkoxy substituiertes Phenyl oder $C_7$- bis $C_9$-Phenylalkyl,

$R^3$ und $R^4$ unabhängig voneinander für Wasserstoff, $C_1$- bis $C_{18}$-Alkyl, durch Sauerstoff ein- oder mehrfach unterbrochenes $C_3$- bis $C_{18}$-Alkyl, $C_1$- bis $C_{18}$-Alkoxy, durch Sauerstoff ein- oder mehrfach unterbrochenes $C_4$- bis $C_{18}$-Alkoxy, gegebenenfalls durch $C_1$- bis $C_4$-Alkyl oder $C_1$- bis $C_4$-Alkoxy substituiertes Phenyl, Phenyl-$C_1$-$C_8$-alkoxy, $C_1$- bis $C_{12}$-C-Acylamino, Benzoylamino, $C_2$-bis $C_{12}$-C-Acyloxy oder Benzoyloxy,

n für 0, 1, 2, 3, 4 oder 5,

X für -O-, 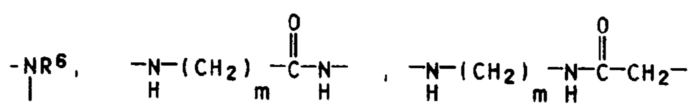 oder

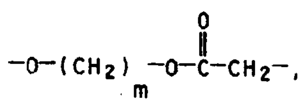

$R^5$ für Wasserstoff, $C_1$- bis $C_8$-Alkyl, $C_7$- bis $C_{10}$-Phenylalkyl, Cyanmethyl, Aminoethyl, Hydroxyethyl, $C_1$- bis $C_8$-C-Acyl oder Benzoyl,

$R^6$ für Wasserstoff, $C_1$- bis $C_8$-Alkyl, $C_7$- bis $C_{10}$-Phenylalkyl oder Phenyl und

m für eine Zahl von 2 bis 8 stehen,

sowie die Säureadditionsalze und Hydrate dieser Verbindungen.

2. Benzimidazolcarbonsäureanilide gemäß Anspruch 1, dadurch gekennzeichnet, daß $R^1$ und $R^2$ Wasserstoff bedeuten.

3. Benzimidazolcarbonsäureanilide gemäß Anspruch 1 oder 2, dadurch gekennzeichnet, daß $R^3$ für Wasserstoff und $R^4$ für Wasserstoff oder Ethoxy stehen.

4. Benzimidazolcarbonsäureanilide gemäß Anspruch 1, 2 oder 3, dadurch gekennzeichnet, daß n 1 oder 2 ist.

5. Benzimidazolcarbonsäureanilide gemäß Anspruch 1, 2, 3 oder 4, dadurch gekennzeichnet, daß X für >NH oder -NH-$CH_2$-$CH_2$-CO-NH- steht.

6. Benzimidazolcarbonsäureanilide gemäß den Ansprüchen 1 bis 5, dadurch gekennzeichnet, daß $R^5$ für Methyl oder Wasserstoff steht.

7. Benzimidazolcarbonsäureanilide gemäß den Ansprüchen 1 bis 5, dadurch gekennzeichnet, daß $R^5$ Wasserstoff ist.

8. Verwendung von Verbindungen gemäß den Ansprüchen 1 bis 7 zum Stabilisieren von organischem Material.

9. Verwendung von Verbindungen gemäß den Ansprüchen 1 bis 7 zum Stabilisieren von Kunststoffen.

10. Verwendung von Verbindungen gemäß den Ansprüchen 1 bis 7 zum Stabilisieren von Lacken.

**11.** Verwendung von Verbindungen gemäß den Ansprüchen 1 bis 7 zum Stabilisieren von Polyolefinen.

**12.** Verwendung von Verbindungen gemäß den Ansprüchen 1 bis 7 zum Stabilisieren von Polyurethanen.

## Claims

**1.** A benzimidazolecarboxanilide of the formula (I)

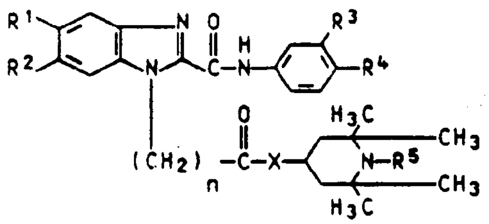

(I) (I)

where $R^1$ and $R^2$ independently of one another are each hydrogen, chlorine, bromine, $C_1$-$C_4$-alkyl, $C_1$-$C_4$-alkoxy, or $C_7$-$C_9$-phenylalkyl or phenyl which is unsubstituted or substituted by $C_1$-$C_4$-alkyl or $C_1$-$C_4$-alkoxy, $R^3$ and $R^4$ independently of one another are each hydrogen, $C_1$-$C_{18}$-alkyl, $C_3$-$C_{18}$-alkyl which is interrupted by one or more oxygen atoms, $C_1$-$C_{18}$-alkoxy, $C_4$-$C_{18}$-alkoxy which is interrupted by one or more oxygen atoms, phenyl which is unsubstituted or substituted by $C_1$-$C_4$-alkyl or $C_1$-$C_4$-alkoxy, or phenyl-$C_1$-$C_8$-alkoxy, $C_1$-$C_{12}$-C-acylamino, benzoylamino, $C_2$-$C_{12}$-C-acyloxy or benzoyloxy, n is 0, 1, 2, 3, 4 or 5,

X is -O-, 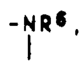, 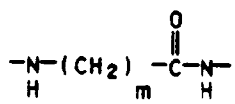, 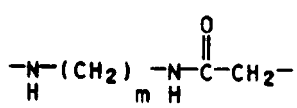 or

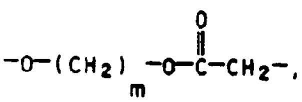,

$R^5$ is hydrogen, $C_1$-$C_8$-alkyl, $C_7$-$C_{10}$-phenylalkyl, cyanomethyl, aminoethyl, hydroxyethyl, $C_1$-$C_8$-C-acyl or benzoyl, $R^6$ is hydrogen, $C_1$-$C_8$-alkyl, $C_7$-$C_{10}$-phenylalkyl or phenyl and m is from 2 to 8, and the acid addition salts and hydrates of this compound.

**2.** A benzimidazolecarboxanilide as claimed in claim 1, wherein $R^1$ and $R^2$ are each hydrogen.

**3.** A benzimidazolecarboxanilide as claimed in claim 1 or 2, wherein $R^3$ is hydrogen and $R^4$ is hydrogen or ethoxy.

**4.** A benzimidazolecarboxanilide as claimed in claim 1, 2 or 3, wherein n is 1 or 2.

**5.** A benzimidazolecarboxanilide as claimed in claim 1, 2, 3 or 4, wherein X is >NH or $-NH-CH_2-CH_2-CO-NH-$.

**6.** A benzimidazolecarboxanilide as claimed in any of claims 1 to 5, wherein $R^5$ is methyl or hydrogen.

**7.** A benzimidazolecarboxanilide as claimed in any of claims 1 to 5, wherein $R^5$ is hydrogen.

**8.** Use of a compound as claimed in any of claims 1 to 7 for stabilizing organic material.

**9.** Use of a compound as claimed in any of claims 1 to 7 for stabilizing plastics.

**10.** Use of a compound as claimed in any of claims 1 to 7 for stabilizing coatings.

**11.** Use of a compound as claimed in any of claims 1 to 7 for stabilizing polyolefins.

**12.** Use of a compound as claimed in any of claims 1 to 7 for stabilizing polyurethanes.

## Revendications

1.- Benzimidazolecarbanilides de formule générale (I)

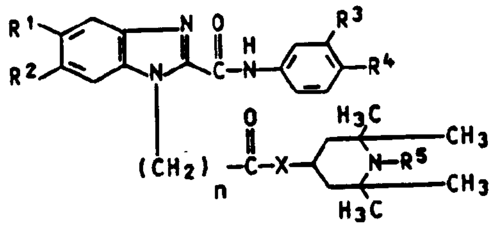

(I),

dans laquelle

$R^1$ et $R^2$ sont mis, indépendamment l'un de l'autre, pour des atomes d'hydrogène, de chlore ou de brome, pour des restes alkyle en $C_1$ à $C_4$, alcoxy en $C_1$ à $C_4$, phényle éventuellement substitué par des radicaux alkyle en $C_1$ à $C_4$ ou alcoxy en $C_1$ à $C_4$, ou pour des restes phénylalkyle en $C_7$ à $C_9$,

$R^3$ et $R^4$ sont mis, indépendamment l'un de l'autre, pour des atomes d'hydrogène, pour des restes alkyle en $C_1$ à $C_{18}$, alkyle en $C_3$ à $C_{18}$ interrompu une ou plusieurs fois par des atomes d'oxygène, alcoxy en $C_1$ à $C_{18}$, alcoxy en $C_4$ à $C_{18}$ interrompu une ou plusieurs fois par des atomes d'oxygène, phényle éventuellement substitué par des radicaux alkyle en $C_1$ à $C_4$ ou alcoxy en $C_1$ à $C_4$, ou pour des restes phényl (alcoxy en $C_1$-$C_8$), acylamino en $C_1$-$C_{12}$, benzoylamino, acyloxy en $C_2$-$C_{12}$ ou benzoyloxy,

n est mis pour 0, 1, 2, 3, 4 ou 5,

$$X \text{ est mis pour } -O-,\ -\underset{|}{N}R^6,\ -\underset{H}{N}-(CH_2)_m-\overset{O}{\overset{\|}{C}}-\underset{H}{N}-,$$

$$-\underset{H}{N}-(CH_2)_m-\underset{H}{N}-\overset{O}{\overset{\|}{C}}-CH_2- \text{ ou } -O-(CH_2)_m-O-\overset{O}{\overset{\|}{C}}-CH_2-,$$

$R^5$ est mis pour un atome d'hydrogène ou pour un reste alkyle en $C_1$ à $C_8$, phénylalkyle en $C_7$ à $C_{10}$, cyanométhyle, aminoéthyle, hydroxyéthyle, acyle en $C_1$ à $C_8$ ou benzoyle,

$R^6$ est mis pour un atome d'hydrogène ou pour un reste alkyle en $C_1$ à $C_8$, phénylalkyle en $C_7$ à $C_{10}$ ou phényle, et

m est mis pour un nombre de 2 à 8.

ainsi que les sels d'addition d'acide et les hydrates de ces composés.

2.- Benzimidazolecarbanilides selon la revendication 1, caractérisés en ce que $R^1$ et $R^2$ représentent des atomes d'hydrogène.

3.- Benzimidazolecarbanilides selon la revendication 1 ou 2, caractérisés en ce que $R^3$ est mis pour un atome d'hydrogène et $R^4$ pour un atome d'hydrogène ou un reste éthoxy.

4.- Benzimidazolecarbanilides selon l'une quelconque des revendications 1 à 3, caractérisés en ce que n est mis pour 1 ou 2.

5.- Benzimidazolecarbanilides selon l'une quelconque des revendications 1 à 4, caractérisés en ce que X est mis pour >NH ou pour $-NH-CH_2-CH_2-CO-NH-$.

6.- Benzimidazolecarbanilides selon l'une quelconque des revendications 1 à 5, caractérisés en ce que $R^5$ est mis pour un reste méthyle ou pour un atome d'hydrogène.

7.- Benzimidazolecarbanilides selon l'une quelconque des revendications 1 à 5, caractérisé en ce que $R^5$ est un atome d'hydrogène.

8.- Utilisation de composés selon l'une quelconque des revendications 1 à 7 pour la stabilisation de matières organiques.

9.- Utilisation de composés selon l'une quelconque des revendications 1 à 7 pour la stabilisation de matières plastiques.

10.- Utilisation de composés selon l'une quelconque des revendications 1 à 7 pour la stabilisation de vernis.

11.- Utilisation de composés selon l'une quelconque des revendications 1 à 7 pour la stabilisation de polyoléfines.

12. - Utilisation de composés selon l'une quelconque des revendications 1 à 7 pour la stabilisation de polyuréthannes.